# EUROPÄISCHE PATENTSCHRIFT

(11) **EP 0 665 209 B1**
(45) Veröffentlichungstag und Bekanntmachung des Hinweises auf die Patenterteilung: **02.12.1998**
(21) Anmeldenummer: 95100901.8
(22) Anmeldetag: 24.01.1995
(51) Int. Cl.: C07C 46/04, C07C 46/06, C07C 50/10, C07C 37/60

(54) **Verfahren zur katalytischen Oxidation von aromatischen Verbindungen**
Process for the catalytic oxidation of aromatic compounds
Procédé d'oxydation catalytique de composés aromatiques

(30) Priorität: 27.01.1994 DE 4402333; 06.06.1994 DE 4419799
(43) Veröffentlichungstag der Anmeldung: 02.08.1995
(73) Patentinhaber: HOECHST AKTIENGESELLSCHAFT, 65929 Frankfurt am Main (DE)
(72) Erfinder: Herrmann, Wolfgang, Anton, Prof. Dr., D-85354 Freising (DE); Galamba Correia, Joao Domingos, D-80796 München (DE); Fischer, Richard Walter, Dr., D-65929 Frankfurt (DE); Adam, Waldemar, Prof. Dr., D-97074 Würzburg (DE); Lin, Jianhua, Dr., Singapur 0512 (SG); Saha-Möller, Chantu Ranjan, Dr., D-97074 Würzburg (DE); Shimizu, Masao, Dr., Ibaraki 305 (JP)

(56) Entgegenhaltungen:
- EP-A- 0 380 085
- EP-A- 0 560 488

## Beschreibung

Zur Oxidation ungesättigter organischer Verbindungen (Olefine, Polyene, Alkine, etc.) sind als Katalysatoren unterschiedlichste, meist binäre Oxide von Übergangsmetallen in der Praxis etabliert. Als Beispiele seien V₂O₅, CrO₃, MoO₃, WO₃, [MnO₄]⁻, OsO₄ und RuO₄ als effiziente Epoxidations-, Hydroxylierungs- bzw. Carboxylierungskatalysatoren genannt (H.A. Jørgensen, Chem. Rev. 1989, 89, S. 431-458).

Der Einsatz solcher Systeme zur katalytischen Oxidation von aromatischen Verbindungen ist allerdings vielen Beschränkungen unterworfen. Fehlende Aktivität (WO₃) einerseits und mangelhafte Selektivität andererseits (CrO₃/H₂SO₄) neben der oft nicht gewährleisteten ökologischen sowie gesundheitlichen bzw. pharmakologischen Unbedenklichkeit (z. B. bei CrO₃ oder OsO₄) verhinderten bislang den technischen Einsatz solcher Katalysatoren.

Andere in der Oxidationschemie etablierte Verfahren, die z. B. mit elektrochemischer Oxidation, Cer(IV)-Salzen, Mangan(III)sulfat oder Persäuren bzw. Peroxiden (t-BuOOH) in Gegenwart von Molybdänkomplexen als Oxidantien arbeiten, erweisen sich bei der Oxidation von einfachen oder kondensierten Aromaten bzw. deren Derivaten als sehr aufwendig, teuer, oft durch den erforderlichen stöchiometrischen Einsatz (Cer(IV)-Salze, Mangan(III)sulfat) mit hohen Salzfrachten belastet und meist auch als unspezifisch (R. P. Kreh et al., J. Org. Chem., 1989, 54, 1526-1531; M. Hudlicky, Oxidations in Organic Chemistry, ACS Monograph 186, Washington/DC, 1990, S. 92-98; T. A. Gorodetskaya et al., U.S.S.R. Patent 1 121 255, 1984; Chem. Abstr., 1985, 102, 203754; W. Adam et al., Synthesis, 1993, 280-282, J. Skarzewski, Tetrahedron, 1984, 40, 4997-5000; S. Yamaguchi et al., Bull. Chem. Soc. Jpn., 1986, 59, 2881-2884; M. Periasamy, M.V. Bhatt, Tetrahedron Lett. 1978, 4561-4562; Y. Asakawa et al., 1988, J. Org. Chem., 53, 5453-5457; W. Chen, Chem. Abstr., 1987, 107, 58620).

Arbeiten von Buchler et al. (DE-A-3731689, DE-A-3731690) haben gezeigt, daß Rhenium-Komplexe Olefine epoxidieren, nicht aber Aromaten.

Aus der EP-A-380085 sind rheniumorganische Verbindungen bekannt, die als Katalysatoren zur Oxidation von Olefinen in Gegenwart von Wasserstoffperoxid eingesetzt werden. Da die Erfahrung gezeigt hat, daß klassische Olefin-Oxidationskatalysatoren zur Oxidation von aromatischen Verbindungen ungeeignet sind, konnte nicht erwartet werden, daß diese rheniumorganischen Verbindungen auch effizient zur Oxidation von Aromaten verwendet werden können.

Chinone und insbesondere Naphthochinon-Derivate sind industriell wertvolle Produkte sowohl zur Weiterverarbeitung (9,10-Anthrachinon ist ein Grundstoff für Bootsfarben) als auch zur unmittelbaren Verwendung, beispielsweise als Vitamine. So stellt 2-Methyl-1,4-naphthochinon als Vitamin K₃ den Grundkörper der Vitamin-K-Gruppe dar. Das Grundgerüst des 2-Methyl-1,4-naphthochinons ist allen fettlöslichen K-Vitaminen gemeinsam, Unterschiede treten allein in den Seitenketten der 3-Position auf. Allein die Direktsynthese des Vitamin K₃ aus der Vorstufe 2-Methylnaphthalin bedeutet eine etwa zehnfache Wertschöpfung. Vitamin-K-Mangel führt zu einer Senkung des Blutspiegels an Gerinnungsfaktoren und damit zu entsprechenden Störungen der Blutgerinnung, was durch Vitamin-K-Gaben wieder behoben werden kann.

Es besteht daher die Aufgabe, ein möglichst leicht zugängliches, einfach handhabbares, lagerfähiges, wirksames Katalysatorsystem zu finden, das die gewünschte Selektivität bei der Oxidation von Aromaten erreicht.

Es wurde nun überraschend gefunden, daß bestimmte rheniumorganische Verbindungen als hochaktive Katalysatoren für die Oxidation von aromatischen Verbindungen, insbesondere für die selektive Überführung in Chinone, geeignet sind, wenn sie zusammen mit peroxidhaltigen Verbindungen in einem flüssigen Medium angewendet werden.

Gegenstand der Erfindung ist ein Verfahren zur Oxidation der allgemeinen Formel I,

R¹ₐRe_{b}O_{c} (I),

welches dadurch gekennzeichnet ist, daß a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3^{.}b ist und worin R¹ gleich oder verschieden ist und einen aliphatischen Kohlenwasserstoffrest mit 1 bis 10 C-Atomen, einen aromatischen Kohlenwasserstoffrest mit 6 bis 10 C-Atomen oder einen Arylalkylrest mit 7 bis 9 C-Atomen darstellt, wobei die Reste R¹ gegebenenfalls unabhängig voneinander gleich oder verschieden substituiert sein können, und bei σ-gebundenen Resten an das C-Atom in α-Stellung wenigstens noch ein Wasserstoffatom gebunden ist, als Katalysatoren zur Oxidaton von elektronenreichen aromatischen Verbindungen sowie deren Derivaten.

Die Verbindungen der allgemeinen Formel I können auch in Form ihrer Lewis-Basen-Addukte vorliegen.

Unter einem aliphatischen Kohlenwasserstoffrest sind Alkylreste mit 1 bis 10 C-Atomen, Alkenyl- oder Alkinylreste mit 2 bis 10 C-Atomen, Cycloalkyl oder Cycloalkenyl mit 3 bis 10 C-Atomen zu verstehen.

Geeignet sind Alkylreste R¹ wie Methyl, Ethyl, Propyl, Isopropyl und die verschiedenen Butyl-, Pentyl-, Hexyl-, Octylreste wie Ethylhexyl- und Decylreste sowie Alkenylreste wie Allyl; geeignet sind auch Cycloalkylreste wie Cyclopropyl, Cyclobutyl, Cyclopentyl, Cyclohexyl, alkyliertes Cyclohexyl wie hydriertes Toluyl, Xylyl, Ethylphenyl, Cumyl oder Cymyl, 1-Menthyl und 1-Norbornyl sowie Alkenylreste wie Vinyl und Allyl und Cycloalkenylreste wie Cyclopentadienyl und Pentamethylcyclopentadienyl. Methyl ist besonders bevorzugt.

Geeignete Arylreste R¹ sind beispielsweise Phenyl oder Naphthyl. Als Beispiel für einen Arylalkylrest sei Benzyl genannt.

Während die Alkyl-, Cycloalkyl- und Arylalkylreste R¹ stets σ-gebunden an das Re-Zentralatom sind, können die Alkenyl-, Alkinyl-, Cycloalkenyl- und Arylreste R¹ σ- oder π-gebunden an das Re-Zentralatom sein.

Der Rest R¹ kann beispielsweise durch Fluor, Chlor, Brom, NH₂, NHR², NR²₂, PH₃, PHR²₂, PH₂R², PR²₃, OH oder OR² substituiert sein, wobei R² gleich oder verschieden ist und einen Alkylrest mit 1 bis 10 C-Atomen oder einen Arylrest mit 6 bis 10 C-Atomen darstellt.

Ein typisches Beispiel für ein Lewis-Basen-Addukt von Verbindungen der Formel I ist CH₃ReO₃·Bipyridin.

Aus sterischen Gründen ist es günstig, wenn die Verbindung der Formel I nicht mehr als drei Gruppen mit mehr als 6 C-Atomen je Rheniumatom trägt; zweckmäßig enthalten die Verbindungen nur eine solche Gruppe.

Bevorzugt sind C₁-C₃-Alkyltrioxorheniumkomplexe, insbesondere Methyltrioxorhenium.

Geeignete Arylverbindungen für das erfindungsgemäße Verfahren sind elektronenreiche aromatische Verbindungen oder kondensierte aromatische Systeme mit 6 bis 22 C-Atomen, bevorzugt mit 6 bis 14 C-Atomen, die gegebenenfalls ein- oder mehrfach, gleich oder verschieden mit einer Elektronendonatorgruppe substituiert sein können. Typische geeignete Elektronendonatorgruppen sind Hydroxy, C₁-C₃-Alkoxy, N-Acylamino, N-Acylamino-C₁-C₃-alkyl, Acyloxy- und C₁-C₃-Alkyl.

Beispiele für solche Arylverbindungen sind Xylole, zwei-, drei- oder vierfach substituierte C₁-C₃-Alkylbenzole oder C₁-C₃-Alkoxybenzole, Naphthalin und seine ein- bis sechsfach substituierten C₁-C₃-Alkyl- oder C₁-C₃-Alkoxyderivate, Anthracen und seine C₁-C₃-Alkyl- oder C₁-C₃-Alkoxyderivate, Phenanthren und höher kondensierte Aromaten, Phenol, Hydrochinon, Resorcin, Brenzcatechin und Pyrogallol, aber auch Biphenyl.

Bevorzugte Arylverbindungen sind Naphthalin und Anthracen sowie ihre Derivate, besonders bevorzugt ist Naphthalin und seine Derivate, insbesondere 2-Methylnaphthalin.

Durch das erfindungsgemäße Verfahren werden die Arylverbindungen im allgemeinen zu den entsprechenden chinoiden Systemen oxidiert. Aus 2-Methylnaphthalin erhält man beispielsweise das 2-Methyl-1,4-naphthochinon, den Grundkörper der Vitamin-K-Reihe.

Bei höhersubstituierten (dreifach und mehr) Arylverbindungen bei denen die Ausbildung eines chinoiden Systems nicht möglich ist, wird durch das erfindungsgemäße Verfahren die entsprechende Hydroxylverbindung hergestellt.

Typische Beispiele für solche höhersubstituierten Arylverbindungen sind 1,2,3,5,8-Pentamethylnaphthalin; 1,2,3-Trimethylbenzol, Mesitylen und 1,3,5-Trimethoxybenzol. Aus diesen Ausgangsstoffen erhält man beispielsweise nach dem erfinderischen Verfahren folgende Hydroxylverbindungen: 4-Hydroxy-1,2,3,5,8-pentamethyl-naphthalin, 1-Hydroxy-3,4,5-trimethylbenzol, 1-Hydroxy-2,4,6-trimethylbenzol und 1-Hydroxy-2,4,6-trimethoxybenzol.

Nach dem erfindungsgemäßen Verfahren wird die zu oxidierende aromatische Verbindung in einem organischen Lösemittel gelöst und mit dem Katalysator versetzt.
Die Konzentration der gelösten aromatischen Verbindung beträgt 0,1 mol in 10-1000 ml, bevorzugt 0,1 mol in 25-250 ml, besonders bevorzugt 0,1 mol in 50-200 ml Lösemittel. Geeignete organische Lösemittel sind beispielsweise Eisessig, THF, tert.-Butanol oder tert.-Butylmethylether, vorzugsweise Eisessig oder THF. Der Katalysator kann in einer Menge von 0,01-10,0 Mol-%, vorzugsweise 0,1-2,0 Mol-%, eingesetzt werden. Zu dieser Lösung wird die peroxidhaltige Verbindung (5-90 Gew.-%) in einem molaren Verhältnis von 1:1 bis zu 20:1 in bezug auf die zu oxidierenden aromatischen Verbindungen gegeben.
Die Reaktionsmischung wird bis zum vollständigen Umsatz bei einer Temperatur von 10-100° C, vorzugsweise 20-60° C, gerührt.
Danach wird die Reaktionsmischung in für den Fachmann üblicher Weise aufgearbeitet, d. h. beispielsweise neutralisiert, extrahiert und getrocknet. Das Rohoxidationsprodukt kann beispielsweise durch Hochvakuumdestillation oder durch Umkristallisation weiter gereinigt werden.

Die rheniumorganischen Verbindungen der Formel I sind bekannt (W.A. Herrmann et al., Angew. Chem. 100 (1988), 420-422; EP-A-380085), ihre Eignung als Oxidationskatalysator von Aromaten ist jedoch neu und war keinesfalls zu erwarten. Sie sind vielmehr die ersten Verbindungen des Rheniums überhaupt, die für die Oxidation von Aromaten erfolgreich eingesetzt werden können. Wegen ihrer Löslichkeitseigenschaften eignen sie sich vorzüglich als Homogenkatalysatoren. Ihr besonderer Vorteil liegt auch darin, daß sie auf einfache Weise aus handelsüblichem Re₂O₇ mit Hilfe gängiger, als Überträger von organischen Gruppen wirkender Substanzen synthetisiert werden können, z. B. im Falle von R¹ = CH₃ durch Umsetzung mit handelsüblichem Tetramethylzinn oder handelsüblichem Dimethylzink. Sie sind gegenüber Luft und Feuchtigkeit, Wasser und Säure unempfindlich, bei Raumtemperatur lagerfähig und in Kombination mit peroxidhaltigen Verbindungen wie Wasserstoffperoxid, anorganischen Peroxiden wie Alkaliperoxiden, insbesondere Natriumperoxid, sowie Percarbonsäuren und ihren Salzen wie m-Chlorperbenzoesäure, Peressigsäure und Magnesiummonoperoxophthalat hochaktive Katalysatoren für die erfindungsgemäßen Oxidationen. Bevorzugt eingesetzt werden die Verbindungen der Formel I in Kombination mit Wasserstoffperoxid.

### Beispiele

### Allgemeine Arbeitsvorschrift zur rheniumkatalysierten Oxidation von aromatischen Verbindungen

Die zu oxidierenden Substrate wurden in Eisessig oder THF gelöst und mit dem Katalysator versetzt. Als letztes wurde Wasserstoffperoxid als Oxidationsmittel zugegeben. Die Reaktionsmischung wurde bis zum vollständigen Umsatz bei 20, 40 bzw. 60° C (s. Tabelle) gerührt.

### Aufarbeitung:

Die Reaktionslösung wurde mit einer gesättigten Natriumhydrogencarbonat-Lösung neutralisiert. Die wäßrige Mutterlauge wurde dreimal mit Methylenchlorid extrahiert, die vereinigten Extrakte wurden über MgSO₄ getrocknet. Das Lösungsmittel wurde dann im Vakuum entfernt. Nach Entfernung des Methylenchlorides wurden im allgemeinen gelb gefärbte, feste Oxidationsprodukte erhalten. Die gemäß obiger Arbeitsvorschrift durchgeführten Beispiele sind Tabelle 1 zu entnehmen. Bei Beispiel Nr. 2 handelt es sich um ein Vergleichsbeispiel.

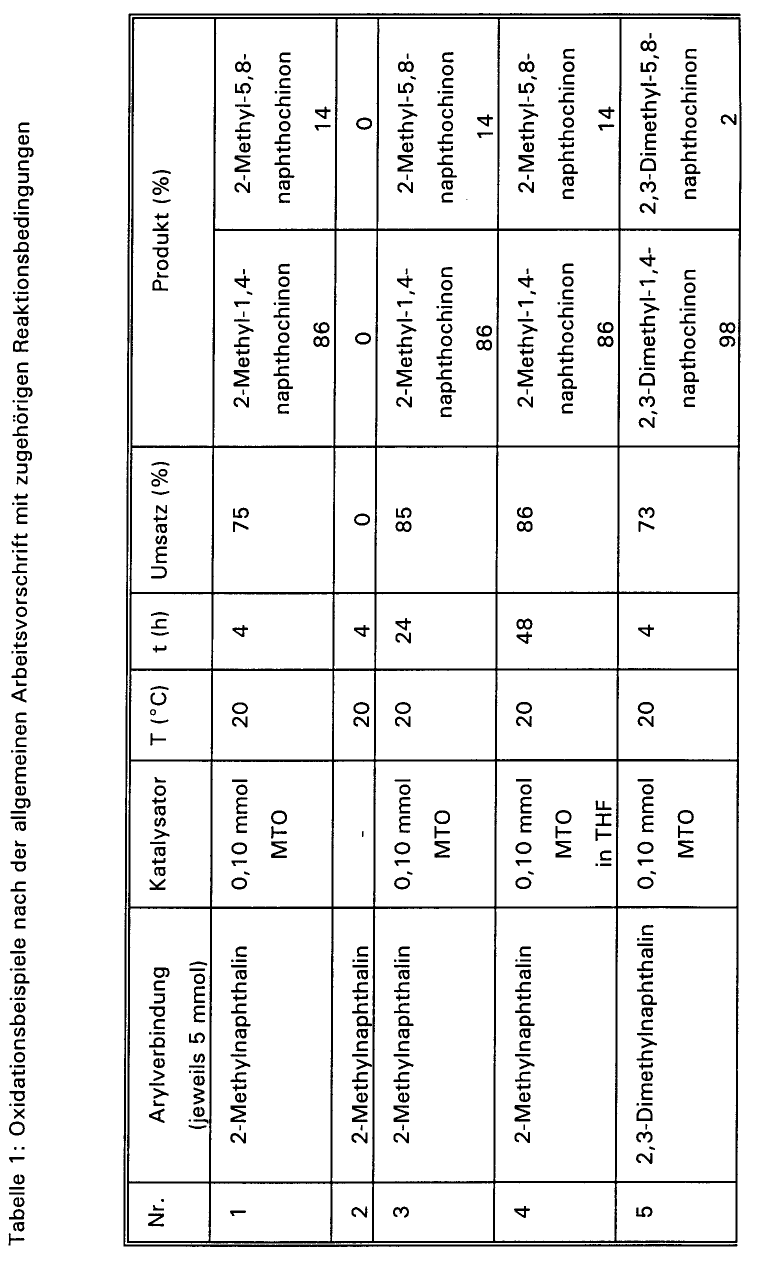

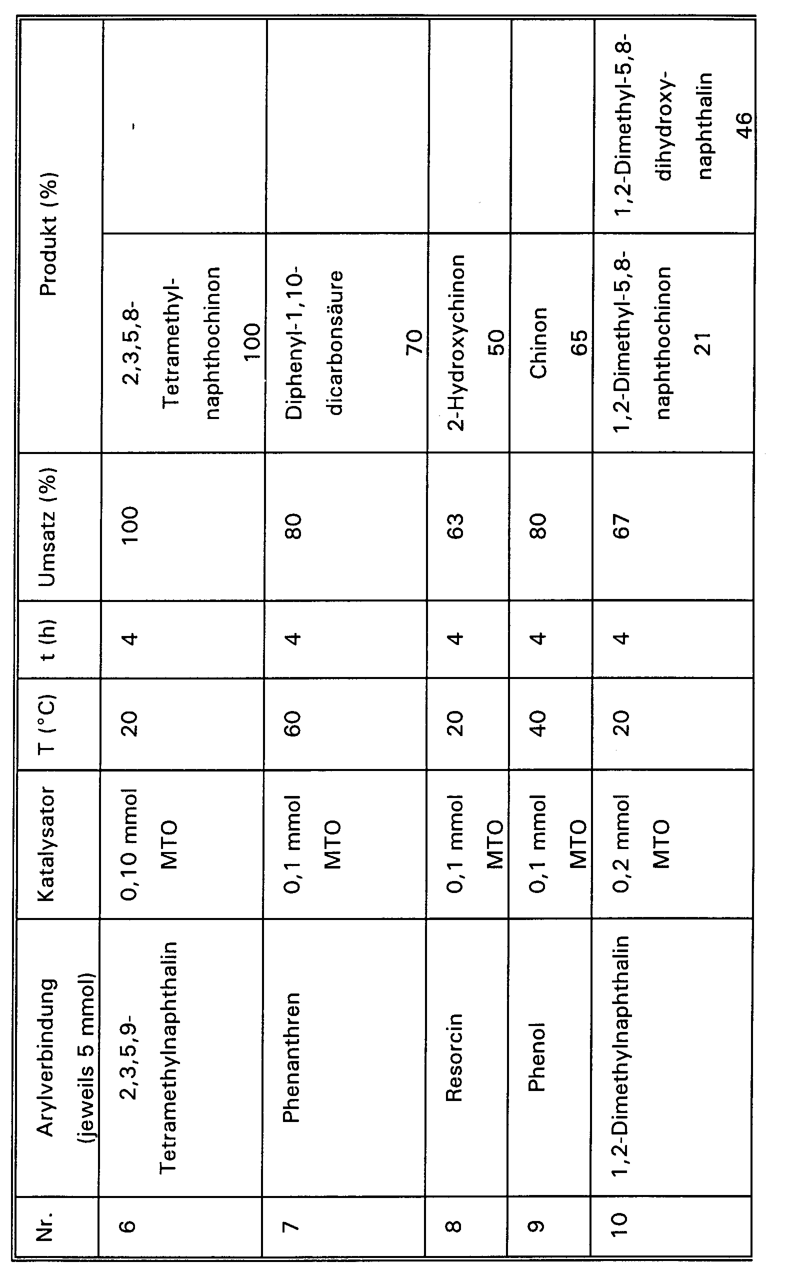

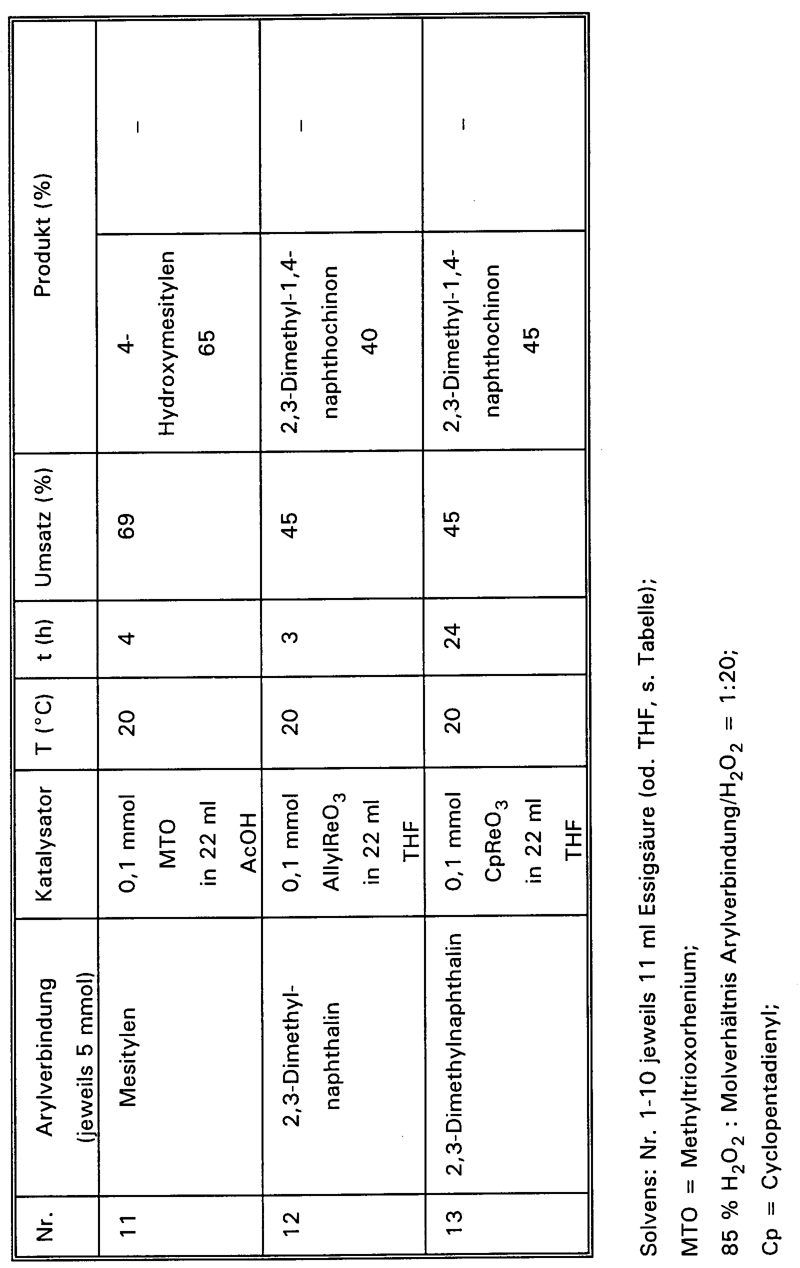

## Patentansprüche

1. Verfahren zur Oxidation von elektronenreichen aromatischen Verbindungen, dadurch gekennzeichnet, daß die elektronenreichen aromatischen Verbindungen mit 6 bis 22 C-Atomen, die ein- oder mehrfach, gleich oder verschieden mit einer Elektronendonatorgruppe, wie Hydroxy, C₁-C₃-Alkoxy, N-Acylamino, N-Acylamino-C₁-C₃-alkyl, Acyloxy- und C₁-C₃-Alkyl, substituiert sind, und deren Derivate, in Gegenwart von einem Katalysator der Formel I
R¹ₐRe_{b}O_{c} (I),
worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3^{.}b ist und worin R¹ gleich oder verschieden ist und einen aliphatischen Kohlenwasserstoffrest mit 1 bis 10 C-Atomen, einen aromatischen Kohlenwasserstoffrest mit 6 bis 10 C-Atomen, oder einen Arylalkylrest mit 7 bis 9 C-Atomen darstellt, wobei die Reste R¹ gegebenenfalls unabhängig voneinander gleich oder verschieden substituiert sein können, und bei σ-gebundenen Resten an das C-Atom in α-Stellung wenigstens noch ein Wasserstoffatom gebunden ist, und einer peroxidhaltigen Verbindung oxidiert werden.

2. Verfahren gemäß Anspruch 3, dadurch gekennzeichnet, daß als peroxidhaltige Verbindung Wasserstoffperoxid eingesetzt wird.

3. Verfahren gemäß Anspruch 3 oder 4, dadurch gekennzeichnet, daß eine C₆-C₁₄-Arylverbindung oxidiert wird.

4. Verfahren gemäß Anspruch 3, 4 oder 5, dadurch gekennzeichnet, daß Naphthalin und seine Derivate oxidiert werden.

5. Verfahren gemäß Anspruch 6, dadurch gekennzeichnet, daß 2-Methylnaphthalin oxidiert wird.

6. Verfahren gemäß Anspruch 1, dadurch gekennzeichnet, daß R¹ C₁-C₃-Alkyl, a 1, b 1 und c 3 ist.

## Claims

1. A process for the oxidation of electron-rich aromatic compounds, which comprises oxidizing the electron-rich aromatic compounds having from 6 to 22 carbon atoms which are monosubstituted or polysubstituted, identically or differently, by an electron-donating group, such as hydroxyl, C₁-C₃-alkoxy, N-acylamino, N-acylamino-C₁-C₃-alkyl, acyloxy and C₁-C₃-alkyl, and their derivatives, in the presence of a catalyst of the formula I
R¹ₐRe_{b}O_{c} (I),
where a is from 1 to 6, b is from 1 to 4 and c is from 1 to 14 and the sum of a, b and c is in accordance with the valence of from 5 to 7 of the rhenium, with the proviso that c is not greater than 3·b, and where R¹ is identical or different and is an aliphatic hydrocarbon radical having from 1 to 10 carbon atoms, an aromatic hydrocarbon radical having from 6 to 10 carbon atoms or an arylalkyl radical having from 7 to 9 carbon atoms, with the radicals R¹ being able, if desired, to be identically or differently substituted independently of one another and, in the case of σ-bonded radicals, at least one hydrogen atom still being bonded to the carbon atom in the α position, and a peroxide-containing compound.

2. The process as claimed in claim 1, wherein the peroxide-containing compound used is hydrogen peroxide.

3. The process as claimed in claim 1 or 2, wherein a C₆-C₁₄-aryl compound is oxidized.

4. The process as claimed in claim 1, 2 or 3, wherein naphthalene or one of its derivatives is oxidized.

5. The process as claimed in claim 4, wherein 2-methylnaphthalene is oxidized.

6. The process as claimed in claim 1, wherein R¹ is C₁-C₃-alkyl, a is 1, b is 1 and c is 3.

## Revendications

1. Procédé d'oxydation de composés aromatiques riches en électrons, caractérisé en ce que l'on oxyde les composés aromatiques riches en électrons avec de 6 à 22 atomes de carbone, qui sont substitués une ou plusieurs fois un groupe électrodonneur identique ou différent, comme les groupes hydroxy, alcoxy en C₁-C₃, N-acylamino, N-acylamino-(alkyle en C₁-C₃), acyloxy et un groupe alkyle en C₁-C₃, et leurs dérivés, en présence d'un catalyseur de formule I
R¹ₐRe_{b}O_{c} (I)
où a va de 1 à 6, b va de 1 à 4 et c va de 1 à 14 et la somme de a, b et c doit satisfaire à une valence de 5 à 7 de rhénium, à la condition que c ne soit pas supérieur à 3.b, et où R¹ est identique ou différent et représente un reste hydrocarboné aliphatique avec de 1 à 10 atomes de carbone, un reste hydrocarboné aromatique avec de 6 à 10 atomes de carbone ou un reste aryle avec de 7 à 9 atomes de carbone, les restes R¹ pouvant éventuellement être substitués, indépendamment l'un de l'autre, par des substituants identiques ou différents, et pour les restes liés par une liaison s aux atones de carbone au moins un atome d'hydrogène est lié en position a, ainsi qu'en présence d'un composé peroxydique.

2. Procédé selon la revendication 3, caractérisé en ce que l'on utilise comme composé peroxydique le peroxyde d'hydrogène.

3. Procédé selon la revendication 3 ou 4 caractérisé en ce que l'on oxyde un composé aryle en C₆-C₁₄.

4. Procédé selon la revendication 3 ou 4 ou 5 caractérisé en qu'on oxyde le naphtalène et ses dérivés.

5. Procédé selon la revendication 6, caractérisé en ce qu'on oxyde le 2-méthylnaphtalène.

6. Procédé selon la revendication 1, caractérisé en ce que R¹ représente un groupe alkyle en C₁-C₃, a vaut 1, b vaut 1 et c vaut 3.
